# FASCICULE DE BREVET EUROPEEN

(11) **EP 1 277 474 B1**
(45) Date de publication et mention de la délivrance du brevet: **02.08.2006**
(21) Numéro de dépôt: 02291786.8
(22) Date de dépôt: 16.07.2002
(51) Int. Cl.: A61K 36/534, A61P 17/00, A61P 21/00

(54) **Association arnica, ruscus et menthol**
Kombination von Arnica, Ruscus und Menthol
Combination of arnica, ruscus and menthol

(30) Priorité: 17.07.2001 FR 0109524
(43) Date de publication de la demande: 22.01.2003
(73) Titulaire: PIERRE FABRE MEDICAMENT, 92100 Boulogne (FR)
(72) Inventeur: Fabre, Pierre, 81106 Castres (FR); Przybylski, Christophe, 31290 Villefranche de Lauragua (FR); Jeanjean, Michel, 31320 Castenet Tolosan (FR)
(74) Mandataire: Warcoin, Jacques

(56) Documents cités:
- WO-A-98/05294

## Description

L'objet de la présente invention est une préparation à visée médicale utilisée pour la prévention ou le traitement des traumatismes de la vie courante et des lésions aiguës du sportif.

Cette préparation est obtenue par l'association de trois extraits végétaux d'Arnica, de Ruscus et de menthe ; elle est appliquée par voie topique sur la zone du corps à traiter et peut se présenter sous une forme adaptée (telle qu'un liniment, une crème, un gel ou un patch).

Les accidents quotidiens de la vie courante (chutes, chocs, petits traumatismes) ou plus graves souvent, les lésions aiguës du sportif, se caractérisent par l'apparition d'ecchymoses, de contusions, d'hématomes et d'oedèmes qui se compliquent parfois de lésions articulaires (entorses, élongations, foulures...).

Ces symptômes sont traités par l'application d'eau glacée ou de substances réfrigérantes qui permettent de réduire l'épanchement tissulaire.

Un autre moyen traditionnel consiste à appliquer des compresses ou des crèmes ou des gels à base d'Arnica, en raison de ses propriétés vulnéraires reconnues. Dénommée autrefois Panacea Lapsorum (la panacée des chutes), l'Arnica fait partie des espèces vulnéraires adminstrées par voie orale, telles que les cite le Codex de 1949 : Absinthe, Bétoine, Calament, Germandrée, Hysope, Lierre terrestre, Origan, Pervenche, Romarin, Sauge, Scolapendre, Scordium, Thym, Véronique, Pied de chat, Tussilage. Elle est surtout largement utilisée par voie topique dans cette même indication, et cet usage local est le seul qui persiste aujourd'hui en raison du risque de toxicité lié à l'absorption orale du produit (exception faite des applications homéopathiques).

Arnica Montana est une plante appartenant à la famille des composées radiées. C'est une espèce des régions montagneuses.

Plante herbacée vivace pouvant atteindre 70 cm, elle présente une tige florifère dressée, et à sa base des feuilles ovales groupées en rosette, et légèrement velues. Les inflorescences sont des capitules solitaires ou groupés. Les fleurs sont toutes d'un jaune orangé. Les fruits sont des akènes surmontés d'une aigrette de poils.

La drogue est constituée par les capitules floraux entiers. Elle est inscrite à la Pharmacopée Française.

Ses principaux constituants sont des triterpènes (arnidiol et faradiol), des lactones sesquiterpéniques (hélénaline et dihydrohélénaline), des flucosides flavoniques (stragaline et isoquercétine), et des substances diverses (choline, polyphénols, acides gras, caroténoïdes).

Mais la fraction responsable de son activité, reste encore inconnue.

La forme la plus couramment utilisée est la teinture, diluée dans l'eau ou dans un alcool de faible titre ; elle est appliquée en compresses sur les contusions, les ecchymoses et autres meurtrissures .

A des concentrations élevées, excédant 4 % environ, il peut apparaître de l'hyperémie, de l'inflammation, des démangeaisons douloureuses et parfois des phlyctènes. Dans tous les cas, l'application à proximité des yeux est exclue. Ces effets secondaires limitent la concentration des préparations à base d'Arnica.

Aussi, les inventeurs ont-ils cherché à potentialiser l'action de l'Arnica, en associant d'autres extraits ou d'autres actifs. Ils ont trouvé deux extraits qui, de façon surprenante, renforcent l'activité de l'Arnica, et l'élève à un niveau que n'atteint aucune autre association déjà décrite avec d'autres actifs tels que l'Origan, la Mélisse, l'Angélique, la Myrrhe, le Gingembre, la Cannelle ou la térébenthine.

Ce résultat surprenant a été obtenu par association de l'Arnica avec un extrait de menthe et un extrait de Ruscus.

Les plantes appartenant au genre MENTHA, correspondent à de nombreuses espèces ; elles présentent de nombreuses variations morphologiques et résultent en outre de fréquentes hybridations entre les espèces, rendant leur classification difficile.

C'est ainsi que la plus connue et la plus largement utilisée, la Menthe poivrée (Mentha piperita L.) résulte de l'hybridation de Mentha aquatica L. et de Mentha spicata L. ; et 'il existe plusieurs variétés de Menthe poivrée.

C'est la seule espèce inscrite à la Pharmacopée Française pour ses sommités fleuries et ses feuilles qui constituent la drogue usuelle. Mais d'autres espèces sont également utilisées dans le domaine pharmaceutique : Menthe douce (ou nahnah), Menthe aquatique, Menthe poulist.

Elles appartiennent toutes à la famille botanique des Labiées. Ce sont des plantes herbacées à tige quadrangulaire de 50 à 120 cm de hauteur, à souche à stolons. Elles font l'objet d'une culture industrielle ou artisanale notamment aux Etats-Unis et en Europe Occidentale. Leur exploitation vise essentiellement l'obtention de l'huile essentielle qui représente 1 à 3 % de la drogue sèche ; elle est obtenue par entrainement à la vapeur d'eau de sommités fleuries récemment cueillies. 500 kg de plante fournit 1 kg environ d'huile essentielle.

Les propriétés analgésiques des Menthes étaient appréciées des anciens déjà qui préconisaient l'application de leur suc (extrait par pression des feuilles) sur les tempes pour calmer les maux de tête. De même le menthol est utilisé de nos jours dans les migraines, les névralgies et les démangeaisons. L'action analgésiante du menthol fut découvert à la suite des travaux de HENSEL et ZOTTERMAN (1951), à propos de l'effet de fraîcheur du menthol sur les récepteurs thermiques de la langue.

Le menthol, lorsqu'il est appliqué localement est un rubéfiant. Son action s'exerce sur les terminaisons nerveuses de la peau, provoquant une sensation de froid par stimulation des récepteurs de froid ; il causerait la dépolarisation des récepteurs et accroîtrait la décharge nerveuse en inhibant l'écoulement du calcium.

C'est cette stimulation des récepteurs de froid qui diminue la perception de la douleur et permet d'interpréter ses propriétés anti-inflammatoires et analgésiques.

Ainsi la Menthe, ou le plus souvent le menthol qui constitue son actif essentiel ont été utilisés seuls ou en association avec divers autres actifs :
- l'essence de Wintergreen ou d'autres dérivés salicylés pour le traitement local des douleurs rhumatismales, avec le camphre, le thymol ou l'essence d'Eucalyptus.
- le célèbre Baume du Tigre associe menthol, camphre, huile essentielle de Girofle, Menthe, Cajeput.

Il est à noter que le menthol peut aussi être obtenu par synthèse, par hydrogénation catalytique du thymol : quelle que soit son origine, le menthol donne lieu aux mêmes usages thérapeutiques.

Dans le cadre de la présente invention, l'association de l'Arnica et de la Menthe permet d'accroître l'efficacité de l'Arnica, puisqu'elle ajoute à son activité propre vis-à-vis des contusions, des hématomes ou des foulures un effet antalgique qui consiste à réduire la perception douloureuse liée au traumatisme.

La demande WO 00/45796 décrit une composition topique contenant 10 % en poids d'un extrait d'Arnica Montana en association avec 10 % de mnethol, du camphre, de l'essence de menthe, de l'essence d'eucalyptus et de la lidocaïne dans un organogel ou du petrolatum. Cette composition est utile dans le traitement de la douleur centrale et périphérique ; elle réduit les oedèmes et les inflammations. Selon l'enseignement de ce document l'association de l'Arnica avec ces analgésiques augmente le flux capillaire de la zone à traiter et facilite ainsi l'absorption des actifs.

Par ailleurs, la demande FR 2 504 009 propose une composition pour le traitement topique de divers troubles de caractère généralement inflammatoire et/ou infectieux tels que les maux de dents contenant un extrait d'Arnica, du camphre, du menthol, un agent tensio-actif et un excipient lipo-aqueux. Les proportions de menthol et d'Arnica sont respectivement comprises entre 0,01 à 0,08 g, et 1 à 4 g. Selon l'enseignement de ce document, la composition présente une activité anti-microbienne, une activité anti-inflammatoire, une activité analgésique locale et une activité cicatrisante de telle sorte qu'elle produit rapidement des effets de guérison.

Les concentrations d'essence de Menthe sont avantageusement comprises entre 1 et 30 %, ce qui exprimé en menthol correspond à des concentrations de 0,3 à 10 % en poids de la composition de l'invention.

Il est notoire que cette concentration en menthol doit rester raisonnable puisque des réactions d'intolérance peuvent apparaître. C'est pour cette raison que la concentration a été limitée à un taux maximum de 10 %.

Mais les inventeurs ont voulu optimiser l'activité de la composition objet de ce brevet, en potentialisant de plus l'activité propre de l'Arnica vis-à-vis des effets sur la circulation. Dans cette perspective, l'association avec l'extrait de Ruscus a donné des résultats surprenants.

Ruscus Aculeatus L. est une plante ligneuse vivace par son rhizome ; elle présente des tiges dressées robustes portant des cladodes. La drogue est constituée par le rhizome et les racines.

Le Ruscus dénommé aussi Fragon épineux, ou petit-houx était l'un des constituants du sirop des cinq racines, avec l'asperge, l'ache, le fenouil et le persil ; il est utilisé actuellement pour ses propriétés veinotoniques et vasculo-protectrices.

La potentialisation de l'Arnica par le Ruscus résulte de l'action de la ruscogénine et à la néo-ruscogénine sur la vasoconstriction et la distensibilité des veines. Il en résulte une augmentation de la pression et du débit veineux, une augmentation du débit lymphatique. La composition de l'invention contient de préférence des extraits de Ruscus titrés en Ruscogénines, à des concentrations comprises entre 0,25 % et 10 %, de préférence 0,25 % et 5 % en poids de la composition de l'invention.

L'association Arnica-Ruscus par voie topique permet d'obtenir un niveau d'efficacité surprenant grâce à la synergie d'action anti-exsudatrice, lymphocinétique et anti-inflammatoire de ces deux constituants, vis-à-vis des traumatismes sportifs sévères. Il en résulte une réduction significative de la douleur, que l'addition de Menthe ou de menthol vient renforcer.

Cette association de trois actifs Arnica-Ruscus-Menthe représente donc une solution nouvelle et originale aux problèmes de la réparation -voire de la prévention- des lésions aiguës du sportif: hématomes, entorses, contusions, foulures, élongations. Elle se distingue particulièrement par son niveau élevé d'efficacité, supérieur à celui de l'Arnica seul, alors même que la concentration de ce constituant reste limitée afin d'éviter les réactions topiques que l'on observe à des concentrations plus fortes.

La présente invention a pour objet une composition pharmaceutique pour application topique, utilisée notamment pour le traitement des traumatismes de la vie courante et des lésions aiguës du sportif contenant un extrait d'Arnica, un extrait de Ruscus et un extrait de Menthe. L'extrait d'Arnica est un extrait d'Arnica, l'extrait de Ruscus est un extrait de Ruscus Aculeatus, l'extrait de menthe est un extrait de Mentha piperita, Mentha Aquatica, Mentha Spicata. L'extrait d'Arnica et l'extrait de Ruscus dont de préférence des extraits fluides.

De façon avantageuse, elle contient 1 à 10 parties de l'extrait d'Arnica, 1 à 15 parties de l'extrait de Ruscus et 0,5 à 2 parties de l'extrait de Menthe, les parties étant exprimées en poids. Elle contient 2 à 4 parties de l'extrait d'Arnica, 2 à 12 parties de l'extrait de Ruscus et 0,5 à 1 partie de d'extrait de Menthe.

Elle est de préférence sous la forme d'une lotion, d'un liniment, d'un gel hydro-alcoolique, d'une crème ou d'une pommade. On préfère que l'extrait d'Arnica soit un extrait fluide ou une teinture-mère, l'extrait de Menthe, une huile essentielle, une essence ou le menthol, l'extrait de Ruscus, la ruscogénine ou la néo-ruscogénine.

Elle contient 1 à 30 %, de préférence 0,3 à 10 % en poids d'extrait de Menthe, 0,25 à 10 %, de préférence 0,25 à 5 % en poids d'extrait de Ruscus, et 0,5 à 4 %, de préférence 2,5 à 4 % en poids d'extrait d'Arnica.

On l'utilise pour la fabrication d'un médicament destiné à la prévention ou au traitement des traumatismes de la vie courante et des lésions aiguës du sportif, notamment les hématomes, les entorses, les contusions, les foulures et les élongations.

A titre d'exemples non-limitatifs, nous préciserons quelques-unes des présentations retenues pour véhiculer les formules décrites ci-dessus.
1) **Lotion :** obtenue en diluant le mélange actif avec de l'alcool à 40 %. Elle est appliquée sous forme de compresses.
2) **Gel hydro-alcoolique** : le mélange actif est dilué dans l'excipient composé de :
   - alcool, propylène glycol, polymère carboxy-cinylique triethanolamine et eau purifiée,
   - ou d'alcool, hydroxyéthylcellulose, monoxynol, eau purifiée.
3) **Crème:** le mélange actif est incorporé dans l'excipient composé de : alcool cétylique, paraffine, cire auto-émulsionnable au cétomacrogol 1000, myristate d'isopropyle, sorbitol, nipagine, nipasol, germall 115, eau purifiée.
4) **Pommade :** le mélange actif est dilué dans l'excipient composé de : Emulgade, octyldodecanol, eau purifiée.
5) **Patch :** pour réaliser la forme « patch », un noyau concentré contenant les principes actifs est préparé ; ce noyau est ensuite inclus dans le véhicule particulier qui permet d'aboutir à la forme « patch ».
L'obtention de ce noyau permet de résoudre le problème que pose la solubilité des trois principes actifs associés. En effet, à l'état pur, ces trois produits concentrés ne forment pas un mélange homogène.

A titre d'exemple, nous citerons diverses formules de mélange de ces trois actifs :

### Exemple 1

| | |
|---|---|
| Teinture-mère d'Arnica | 4 parties |
| Huile essentielle de menthe | 12 parties |
| Ruscogénines | 0,5 parties. |

### Exemple 2

| | |
|---|---|
| Teinture-mère d'Arnica | 4 parties |
| Menthol | 8 parties |
| Ruscogénines | 0,5 parties |

### Exemple 3 (pour peaux fragiles et réactives)

| | |
|---|---|
| Extrait fluide d'Arnica | 2,5 parties |
| Menthol | 2,5 parties |
| Ruscogénines | 0,75 parties |

### Exemple 4

A titre d'exemple particulier, nous citerons la formule du gel ci-dessous :
- teinture d'Arnica 4 g
- menthol 3 g
- Ruscogénines 0,5 g

Excipients QSP 100 g : alcool à 96, carbomère 980, propylène glycol, diisopropylamine, eau purifiée.

Ce gel a été utilisé pour démontrer l'activité de la préparation faisant l'objet de ce brevet.

Il a été appliqué à un groupe de 22 individus.

Un groupe témoin de 20 individus recevait un gel de référence contenant pour actif la teinture mère d'Arnica seule.

Les quarante deux patients présentaient tous un traumatisme, ou hématome ou contusion de la cuisse. Etaient exclus les sujets présentant des plaies ouvertes.

Pendant la période de l'évaluation, l'utilisation d'analgésiques et d'anti-inflammatoires était interdite.

Le traitement comportait trois applications/jour de 3 g de gel, sans massage sur la zone traumatisée. Sa durée est de 14 jours. Les critères d'évaluation mesuraient :
- la perte fonctionnelle : force musculaire limite ne déclenchant pas la douleur,
- le gonflement : tour de cuisse,
- la rougeur : température de la peau,
- la douleur au repos, à la pression et au mouvement.

L'évolution de ces différents paramètres est enregistrée à J0, J4, J8, J12 et à la fin au 14^{ème} jour.

La comparaison de l'évolution de ces paramètres, parmi les patients montre une supériorité constante de la formule associant la composition des trois actifs, par rapport à la formule contenant l'Arnica seul. La différence entre les deux groupes est statistiquement significative dès le 6^{ème} jour de traitement, elle s'approfondit jusqu'à la fin du traitement démontrant l'efficacité de la composition active qui permet un rétablissement complet plus rapide.

Il faut noter qu'aucun effet indésirable n'a été signalé.

### Exemple 5 :

Un patch préparé à partir de l'un des noyaux actif est préparé. Le noyau de formule suivante est préparé à partir d'une solution alcoolique :

| | |
|---|---|
| Arnica, Extrait fluide | 14,7 % (p/p) |
| Ruscus, Extrait fluide | 11,8 % (p/p) |
| Menthol | 14,7 % (p/p) |
| Polysorbate® 80 | 23 % (p/p) |
| Alcool 95° | 35,8 % (p/p) |

Ce mélange, après dissolution dans le véhicule spécifique du patch à raison de 17 parties de noyau concentré pour 100 p de produit final appliqué ―permet d'obtenir la concentration active adéquate.

## Revendications

1. Composition pharmaceutique pour application topique, utilisée notamment pour le traitement des traumatismes de la vie courante et des lésions aiguës du sportif contenant un extrait d'Arnica, un extrait de Ruscus et un extrait de Menthe.

2. Composition selon la revendication 1, **caractérisée en ce que** l'extrait d'Arnica est un extrait d'Arnica montana.

3. Composition selon la revendication 1 ou 2, **caractérisée en ce que** l'extrait de Ruscus est un extrait de Ruscus Aculeatus.

4. Composition selon l'une des revendications précédentes, **caractérisée en ce que** l'extrait de menthe est un extrait de Mentha piperita, Mentha aquatica, Mentha spicata.

5. Composition selon l'une des revendications précédentes, **caractérisée en ce qu'**elle contient 1 à 10 parties de l'extrait d'Arnica, 1 à 15 parties de l'extrait de Ruscus et 0,5 à 2 parties de l'extrait de Menthe, les parties étant exprimées en poids.

6. Composition selon la revendication 5, **caractérisée en ce qu'**elle contient 2 à 4 parties de l'extrait d'Arnica, 2 à 12 parties de l'extrait de Ruscus et 0,5 à 1 partie de d'extrait de Menthe.

7. Composition selon l'une des revendications précédentes, **caractérisée en ce qu'**elle est sous la forme d'une lotion, d'un liniment, d'un gel hydro-alcoolique, d'une crème, d'une pommade ou d'un patch.

8. Composition selon l'une des revendications précédentes, **caractérisée en ce que** l'extrait d'Arnica est un extrait fluide ou une teinture-mère.

9. Composition selon l'une des revendications précédentes, **caractérisée en ce que** l'extrait de Menthe est une huile essentielle, une essence ou le menthol.

10. Composition selon l'une des revendications précédentes, **caractérisée en ce que** l'extrait de Ruscus est la ruscogénine ou la néo-ruscogénine.

11. Composition selon l'une des revendications précédentes, **caractérisée en ce qu'**elle contient 1 à 30 %, de préférence 0,3 à 10 % en poids d'extrait de Menthe, 0,25 à 10 %, de préférence 0,25 à 5 % en poids d'extrait de Ruscus et 0,5 à 4 %, de préférence 2,5 à 4 % en poids d'extrait d'Arnica.

12. Composition selon l'une des revendications précédentes, **caractérisée en ce qu'**elle st sous la forme d'un patch contenant les trois extraits formulés dans un noyau.

13. Composition selon la revendication 12, **caractérisée en ce que** le noyau du patch est préparé à partir d'une solution alcoolique contenant 12 à 17 % en poids d'un extrait d'Arnica, 12 à 17 % en poids d'extrait de Menthe et 10 à 15 % en poids d'extrait de Ruscus.

14. Utilisation d'une composition selon l'une des revendications 1 à 12 pour la fabrication d'un médicament destiné à la prévention ou au traitement des traumatismes de la vie courante et des lésions aiguës du sportif, notamment les hématomes, les entorses, les contusions, les foulures et les élongations.

## Claims

1. Pharmaceutical composition for topical application, used in particular for the treatment of traumas of everyday life and of acute lesions in sportsmen and sportswomen, containing an extract of arnica, an extract of ruscus and an extract of mint.

2. Composition according to Claim 1, **characterized in that** the extract of arnica is an extract of *Arnica montana.*

3. Composition according to Claim 1 or 2, **characterized in that** the extract of ruscus is an extract of *Ruscus aculeatus.*

4. Composition according to one of the preceding claims, **characterized in that** the extract of mint is an extract of *Mentha piperita, Mentha aquatica* or *Mentha spicata.*

5. Composition according to one of the preceding claims, **characterized in that** it contains 1 to 10 parts of the extract of arnica, 1 to 15 parts of the extract of ruscus and 0.5 to 2 parts of the extract of mint, the parts being expressed by weight.

6. Composition according to Claim 5, **characterized in that** it contains 2 to 4 parts of the extract of arnica, 2 to 12 parts of the extract of ruscus and 0.5 to 1 part of the extract of mint.

7. Composition according to one of the preceding claims, **characterized in that** it is in the form of a lotion, a liniment, an aqueous-alcoholic gel, a cream, an ointment or a patch.

8. Composition according to one of the preceding claims, **characterized in that** the extract of arnica is a fluid extract or a mother tincture.

9. Composition according to one of the preceding claims, **characterized in that** the extract of mint is an essential oil, an essence or mint.

10. Composition according to one of the preceding claims, **characterized in that** the extract of ruscus is ruscogenin or neoruscogenin.

11. Composition according to one of the preceding claims, **characterized in that** it contains 1 to 30%, preferably 0.3 to 10% by weight of extract of mint, 0.25 to 10%, preferably 0.25 to 5% by weight of extract of ruscus and 0.5 to 4%, preferably 2.5 to 4% by weight of extract of arnica.

12. Composition according to one of the preceding claims, **characterized in that** it is in the form of a patch containing the three extracts formulated in a core.

13. Composition according to Claim 12, **characterized in that** the core of the patch is prepared from an alcoholic solution containing 12 to 17% by weight of an extract of arnica, 12 to 17% by weight of extract of mint and 10 to 15% by weight of extract of ruscus.

14. Use of a composition according to one of Claims 1 to 12, for the manufacture of a medicinal product intended for the prevention or for the treatment of traumas of everyday life and acute lesions in sportsmen and sportswomen, in particular haematomas, twists, contusions, sprains and muscle strains.

## Patentansprüche

1. Pharmazeutische Zusammensetzung für die topische Anwendung, insbesondere für die Behandlung von Traumen des täglichen Lebens und von akuten Sportverletzungen verwendet, die einen Arnika-Extrakt, einen Mäusedorn-Extrakt und einen Minze-Extrakt enthält.

2. Zusammensetzung nach Anspruch 1, **dadurch gekennzeichnet, dass** der Arnika-Extrakt ein Extrakt von Arnica montana ist.

3. Zusammensetzung nach Anspruch 1 oder 2, **dadurch gekennzeichnet, dass** der Mäusedorn-Extrakt ein Extrakt von Ruscus aculeatus ist.

4. Zusammensetzung nach einem der vorangehenden Ansprüche, **dadurch gekennzeichnet, dass** der Minze-Extrakt ein Extrakt von Mentha piperita, Mentha aquatica, Mentha spicata ist.

5. Zusammensetzung nach einem der vorangehenden Ansprüche, **dadurch gekennzeichnet, dass** sie 1 bis 10 Teile des Arnika-Extrakts, 1 bis 15 Teile des Mäusedorn-Extrakts und 0,5 bis 2 Teile des Minze-Extrakts enthält, wobei die Teile in Gewicht ausgedrückt sind.

6. Zusammensetzung nach Anspruch 5, **dadurch gekennzeichnet, dass** sie 2 bis 4 Teile Arnika-Extrakt, 2 bis 12 Teile Mäusedorn-Extrakt und 0,5 bis 1 Teil Minze-Extrakt enthält.

7. Zusammensetzung nach einem der vorangehenden Ansprüche, **dadurch gekennzeichnet, dass** sie in Form einer Lotion, eines Liniments, eines wässrig-alkoholischen Gels, einer Creme, einer Salbe oder eines Pflasters vorliegt.

8. Zusammensetzung nach einem der vorangehenden Ansprüche, **dadurch gekennzeichnet, dass** der Arnika-Extrakt ein flüssiger Extrakt oder eine Mutter-Tinktur ist.

9. Zusammensetzung nach einem der vorangehenden Ansprüche, **dadurch gekennzeichnet, dass** der Minze-Extrakt ein ätherisches Öl, eine Essenz oder Menthol ist.

10. Zusammensetzung nach einem der vorangehenden Ansprüche, **dadurch gekennzeichnet, dass** der Mäusedorn-Extrakt Ruscogenin oder Neoruscogenin ist.

11. Zusammensetzung nach einem der vorangehenden Ansprüche, **dadurch gekennzeichnet, dass** sie 1 bis 30, vorzugsweise 0,3 bis 10 Gew.-% Minze-Extrakt, 0,25 bis 10, vorzugsweise 0,25 bis 5 Gew.-% Mäusedorn-Extrakt und 0,5 bis 4, vorzugsweise 2,5 bis 4 Gew.-% Arnika-Extrakt enthält.

12. Zusammensetzung nach einem der vorangehenden Ansprüche, **dadurch gekennzeichnet, dass** sie in Form eines Pflasters vorliegt, das die drei formulierten Extrakte in einem Kern enthält.

13. Zusammensetzung nach Anspruch 12, **dadurch gekennzeichnet, dass** der Kern des Pflasters ausgehend von einer alkoholischen Lösung hergestellt wird, die 12 bis 17 Gew.-% eines Arnika-Extrakts, 12 bis 17 Gew.-% Minze-Extrakt und 10 bis 15 Gew.-% Mäusedorn-Extrakt enthält.

14. Verwendung einer Zusammensetzung nach einem der Ansprüche 1 bis 12 für die Herstellung eines Medikaments, das zur Verhütung oder Behandlung von Traumen des täglichen Lebens oder von akuten Sportverletzungen, insbesondere Hämatomen, Verstauchungen, Prellungen, Zerrungen und Überdehnungen, bestimmt ist.
